## Europäisches Patentamt
## European Patent Office
## Office européen des brevets

(19)

(11) Publication number: **0 000 805**
**A1**

(12)

# EUROPEAN PATENT APPLICATION

(21) Application number: **78300142.3**

(22) Date of filing: **12.07.78**

(51) Int. Cl.²: **C 07 C 87/60, C 07 C 85/11**

(30) Priority: **13.06.77 GB 34069/77**

(43) Date of publication of application:
**21.02.79 Bulletin 79/4**

(84) Designated contracting states:
**BE DE FR GB NL**

(71) Applicant: **The Clayton Aniline Company Limited**
**P.O. Box 2,**
**Clayton Manchester M11 4AP(GB)**

(72) Inventor: **Hildreth, John David**
**Damson Cottage Andertons Lane**
**Henbury Macclesfield Cheshire(GB)**

(72) Inventor: **Haslam, David Geoffrey**
**72 Church Road**
**Kearsley Bolton Lancashire(GB)**

(74) Representative: **Sharman, Thomas**
**CIBA-GEIGY (UK) Limited Tenax Road**
**Trafford Park Manchester M17 1WT(GB)**

(54) Catalytic hydrogenation process for the manufacture of chlorinated aromatic amines.

(57) A process for the manufacture of chlorinated aromatic amines comprises catalytically hydrogenating the corresponding molten nitro compound in the presence of thiophene as a dechlorination inhibitor.

EP 0 000 805 A1

Croydon Printing Company Ltd.

The present invention relates to the production of chlorinated aromatic amines.

Chlorinated aromatic amines are important starting materials for the manufacture of dyestuffs and pigments by diazotisation followed by coupling with various coupling components. It is known that chlorinated aromatic amines can be prepared by catalytic hydrogenation of the corresponding nitro compound. However, in the normal course of this reaction, some dechlorination also occurs. Attempts have been made to reduce this dechlorination using deactivated catalysts, such as sulphided platinum on carbon, or by deliberately adding a catalyst poison, such as a thioether in suitable amounts. All these reactions have been performed in solution in various solvents.

We have now surprisingly found that molten chlorinated aromatic nitro compounds can be reduced in the melt using a catalyst and a controlled amount of thiophene as dechlorination inhibitor. This gives much greater productivity and avoids the problem of recovering the product from the solvent, which involves expensive distillation. It can be easily separated from the resulting aqueous layer and the catalyst filtered off, or in some cases, after removal of catalyst, the whole of the product mixture including the water can be used intact, e.g. to be diazotised prior to coupling.

Accordingly, the present invention provides a process for the manufacture of chlorinated aromatic amines which comprises catalytically hydrogenating the corresponding molten nitro compound in the presence of thiophene as dechlorination inhibitor.

The catalyst may be any that is known for carrying out catalytic hydrogenation reactions, such as nickel, platinum, palladium, ruthenium and rhodium. We prefer to use platinum on charcoal as the catalyst, in suitable catalytic amounts. It is possible to use already deactivated catalysts as well, if desired, such as sulphided platinum on charcoal. The amount of catalyst may be varied over a wide range and may be from 0.005% to 0.1%, based on the weight of the nitro compound. We prefer to use about 0.01%.

The amount of thiophene used may be from 25% to 300% by weight of the catalyst (pure 100% metal), but preferably from 100 to 250%. If too much thiophene is used, it will poison the catalyst too much, and this has the effect of slowing or even stopping the hydrogenation. If too little thiophene is used, there is insufficient inhibition of the dechlorination.

Although the process of the invention is very effective in preventing dechlorination, a very small amount may still occur, and this will result in the formation of hydrochloric acid in the reactor. In order to avoid acidic liquors being formed, it is preferred to add a small amount of a hydrochloric acid acceptor to the reactants.

so as to maintain a reaction pH of 7.0 - 9.0. For example, a carbonate or bicarbonate of an alkali metal may be added in amounts of up to 5% by weight. Preferably, about 0.1 - 1.0% of sodium carbonate or bicarbonate is added.

The reaction is carried out at a temperature above the melting point of the nitro compound being reduced. Once the nitro compound has been melted it is not necessary to heat to a higher temperature, although this may be done if desired.

The reaction may be carried out under elevated pressure which may be from 15 to 1400 psi, preferably from 70 to 420 psi.

The reaction is continued until consumption of hydrogen ceases, which may take from $1\frac{1}{2}$ to 10 hours. The time taken is dependent on the temperature and pressure used in the reaction and on the efficiency of the mixing during the reaction. For example, reaction times may be reduced by carrying out the hydrogenation in a spray nozzle high circulation reactor, such as a Buss loop reactor. Such a loop reactor normally renders an enhanced selectivity to the catalyst through more efficient mixing and cooling of the exothermic reaction. After uptake of hydrogen ceases, it is preferred to maintain the reaction conditions for an additional time, e.g. up to 30 minutes, preferably about 15 minutes to ensure that the reaction is complete.

Before the hydrogenation is started, the reactor should be purged to remove oxygen. The purging can be carried out using an inert gas, such as nitrogen or, preferably, hydrogen.

The process of the invention can be applied to various chlorinated nitro aromatic compounds. These include, for example, 2-chloro-4-nitro-aniline, 2-chloro-4-nitrotoluene, 2,5-dichlornitrobenzene, 2,4-dichlornitrobenzene, 2,3-dichlornitrobenzene, o-, m- and p-chloro-nitrobenzene and 2,4,5-trichlornitrobenzene.

The invention is illustrated by the following Examples, in which parts by weight bear the same relationship to parts by volume as do kilograms to litres.

Example 1

A shaking autoclave was charged with 258.6 parts by weight of 2-chloro-4-nitroaniline. To this was added 0.51 parts by weight of catalyst comprising 5% sulphided platinum on charcoal, as a 50% paste, 0.5 parts by weight sodium carbonate and 0.06 parts by weight of thiophene. The reactor was then closed, purged twice with hydrogen and then heated to 120°C. to melt the educt. With agitation, hydrogen was introduced at 140 psi and the conditions were maintained for 10 hours when consumption of hydrogen ceased.

The pressure was released and the catalyst filtered off. After drying and cooling the product was obtained in excellent yield and purity (less than 0.2% p-phenylenediamine present as impurity).

Example 2

A shaking autoclave was charged with 150 parts by weight 2-chloro-4-nitrotoluene (containing 0.5% p-nitrotoluene as impurity) and a mixture of 1.5 parts by weight of catalyst comprising 1% platinum on charcoal, as a 50% paste, 1.5 parts by weight of sodium bicarbonate, 5 parts by volume water (to mix the catalyst) and 0.025 parts by volume thiophene.

The mixture was hydrogenated at a pressure of 140 psi and a temperature of 85 - 90°C. Reduction was completed in $2\frac{3}{4}$ hours and the product was obtained in 95.8% yield. The product contained 0.5% p-toluidine as impurity formed entirely from the 0.5% nitrotoluene present in the starting material, i.e. no p-toluidine formed during the reduction by dechlorination of 2-chloro-4-nitrotoluene.

Example 3

To a shaking autoclave was charged 300 parts by weight 2-chloro-4-nitrotoluene (containing only 0.1% p-nitrotoluene as impurity) and a mixture of 3 parts by weight of catalyst comprising 1% platinum on charcoal, as a 50% paste, 3 parts by weight sodium bicarbonate, 5 parts by volume water and 0.05 parts by volume thiophene. The mixture was hydrogenated at a pressure of 210 psi and a temperature of 115° - 120°C. Reduction was complete in $4\frac{1}{2}$ hours. The product was obtained in 96.3% yield and containing only 0.1% p-toluidine as the main impurity.

Example 4

To a shaking autoclave was charged 300 parts by weight, 2,5-dichlornitrobenzene, and a mixture of 3 parts by weight catalyst comprising 1% platinum on charcoal, as a 50% paste, 3 parts by weight sodium bicarbonate, 5 parts by weight water and 0.05 parts by weight thiophene. The mixture was hydrogenated at a pressure of 140 psi and at a temperature of 90 - 95°C. Consumption of hydrogen ceased after 3 hours, but the conditions were maintained for a further 15 minutes. The product was obtained in a yield of 95.6%. After removal of the catalyst by filtration, the entire reaction mass could be retained intact and used, e.g. for diazotisation prior to coupling with the desired coupling component.

What we claim is:

1.  A process for the manufacture of chlorinated aromatic amines by catalytically hydrogenating the corresponding nitro compound characterised in that the molten nitro compound is catalytically hydrogenated in the presence of thiophene as a dechlorination inhibitor.

2.  A process as claimed in claim 1 characterised in that the hydrogenation catalyst is nickel, platinum, palladium, ruthenium or rhodium.

3.  A process as claimed in claim 1 or 2 characterised in that the catalyst is platinum or charcoal, or sulphided platinum on charcoal.

4.  A process as claimed in any preceding claim characterised in that the amount of catalyst is from 0.005% to 0.1% based on the weight of the nitro compound.

5.  A process as claimed in any preceding claim characterised in that the amount of thiophene is from 25% to 300% by weight of the catalyst (pure 100% metal).

6.  A process as claimed in claim 5 characterised in that the amount of thiophene is from 100% to 250% by weight of the catalyst (pure 100% metal).

7.   A process as claimed in any preceding claim characterised in that a hydrochloric acid acceptor is added to the reactants to maintain a reaction pH of 7.0 to 9.0.

8.   A process as claimed in claim 7 characterised in that up to 5% by weight of a carbonate or bicarbonate of an alkali metal is added.

9.   A process as claimed in any preceding claim characterised in that it is carried out at a pressure of from 15 to 1400 psi.

10.  A process as claimed in any preceding claim characterised in that the nitro compound is 2-chloro-4-nitroaniline, 2-chloro-4-nitrotoluene, 2,5-dichlornitrobenzene, 2,4-dichlornitrobenzene, 2,3-dichlornitrobenzene, or o-, -or p-chloro-nitrobenzene or 2,4,5-trichlornitrobenzene.

T. Sharman
Agent for the Applicants

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | C 07 C 87/60 <br> C 07 C 85/11 |
| | FR – A – 2 330 669 (BAYER A.G.) <br><br> * Page 2, lines 13-26; page 4, lines 25-30; page 5, line 1 – page 7, line 17 * <br><br> ——— | 1-10 | |

**TECHNICAL FIELDS SEARCHED (Int.Cl.³)**

C 07 C 85/11

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 10-11-1978 | PAUWELS |

EPO Form 1503.1  06.78